(19) Europäisches Patentamt
European Patent Office
Office européen des brevets

(11) Veröffentlichungsnummer: **0 050 095**
**A1**

(12) # EUROPÄISCHE PATENTANMELDUNG

(21) Anmeldenummer: 81810400.2

(22) Anmeldetag: 05.10.81

(51) Int. Cl.³: C 07 C 161/00
C 07 D 307/32, C 07 D 333/36
A 01 N 41/00, A 01 N 43/08
A 01 N 43/10

(30) Priorität: 09.10.80 CH 7550/80
02.09.81 CH 5650/81

(43) Veröffentlichungstag der Anmeldung:
21.04.82 Patentblatt 82/16

(84) Benannte Vertragsstaaten:
AT BE CH DE FR GB IT LI LU NL SE

(71) Anmelder: CIBA-GEIGY AG
Patentabteilung Postfach
CH-4002 Basel(CH)

(72) Erfinder: Eckhardt, Wolfgang, Dr.
Breslauerstrasse 16
D-7850 Lörrach(DE)

(72) Erfinder: Kunz, Walter, Dr.
Im Goldbrunnen 55
CH-4104 Oberwil(CH)

(72) Erfinder: Riebli, Peter
Dittingerstrasse 12
CH-4053 Basel(CH)

(54) **Thiosulfamoyloxy-acetamid-Derivate als Mikrobizide.**

(57) Thiosulfamoyloxy-acetamid-Derivate der Formel

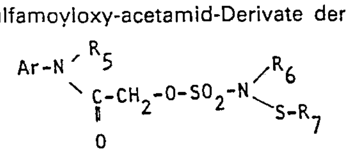

haben ausgezeichnete mikrobizide Wirkungen und eignen sich zur Bekämpfung von Pilzkrankheiten an Kulturpflanzen. In dieser Formel bedeuten

Ar einen Rest der Formeln

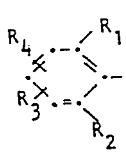

oder

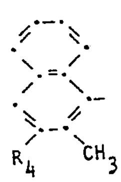

$R_1$ Wasserstoff, Chlor oder Methyl,
$R_2$ Wasserstoff, Chlor, Methyl, Nitro, Amino, Methylamino, Methoxy, Cyano oder Trifluormethyl,
$R_3$ Wasserstoff, Chlor, Methyl, Nitro, Amino, Cyan oder Alkoxymethyl mit nicht mehr als 5 Kohlenstoffatomen,
$R_4$ Wasserstoff oder Methyl,
$R_5$ einen Rest der Formeln

$-CH(CH_3)-CO-X-R_8$ oder

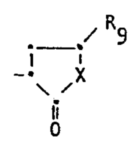

,

$R_6$ $C_1$-$C_4$-Alkyl,
$R_7$ Halo($C_1$-$C_2$-alkyl)
$R_8$ gegebenenfalls durch Halogen oder Methoxy substituiertes $C_1$-$C_3$ Alkyl,
$R_9$ Wasserstoff oder Methyl und
X Sauerstoff oder Schwefel.

EP 0 050 095 A1

CIBA-GEIGY AG
Basel (Schweiz)

5-13093/1+2

# Thiosulfamoyloxy-acetamid-Derivate als Mikrobizide

Die vorliegende Erfindung betrifft mikrobizid wirksame Thiosulfamoyloxy-acetamid-Derivate, Verfahren zu ihrer Herstellung, Mittel, in denen diese als aktive Komponente vorliegen, sowie deren Verwendung zur Bekämpfung von Pilzen und Bakterien auf dem Pflanzenschutzgebiet sowie deren Einsatz im Vorratsschutz.

Die neuen Verbindungen entsprechen der allgemeinen Formel I

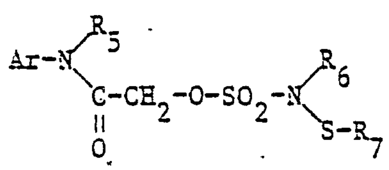

(I)

worin Ar einen Rest der Formeln

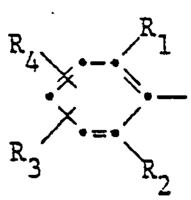

oder

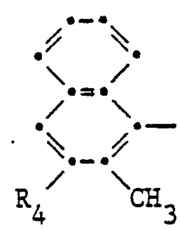

,

$R_1$ Wasserstoff, Chlor oder Methyl,

$R_2$ Wasserstoff, Chlor, Methyl, Nitro, Amino, Methylamino, Methoxy, Cyan oder Trifluormethyl,

$R_3$ Wasserstoff, Chlor, Methyl, Nitro, Amino, Cyan oder Alkoxymethyl mit nicht mehr als 5 Kohlenstoffatomen,

$R_4$ Wasserstoff oder Methyl,

$R_5$ einen Rest der Formeln $-CH(CH_3)-CO-X-R_8$ oder 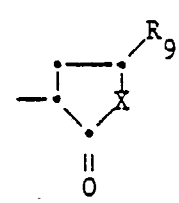 ,

$R_6$ $C_1$-$C_4$-Alkyl,

$R_7$ Halo($C_1$-$C_2$-alkyl),

$R_8$ gegebenenfalls durch Halogen oder Methoxy substituiertes $C_1$-$C_3$-Alkyl,

$R_9$ Wasserstoff oder Methyl und

X Sauerstoff oder Schwefel bedeuten.

Unter Halogen sind in der Definition von $R_8$ Fluor, Chlor, Brom oder Jod, vorzugsweise jedoch Fluor und Chlor zu verstehen.

Der Ausdruck Halo($C_1$-$C_2$-alkyl) soll hier und im folgenden für eine teilweise oder perhalogenierte Methyl- oder Aethylgruppe stehen, beispielsweise für $CF_3$, $CF_2H$, $CFH_2$, $CCl_3$, $CCl_2H$, $CClH_2$, $CFCl_2$, $CF_2Cl$, CFClH, $CBr_3$, $CBr_2H$, $CBrH_2$, $CCl_2Br$, $CClBr_2$, CFClBr, $CF_2CF_3$, $CCl_2CCl_3$, $CBr_2CBr_3$, $CF_2CF_2H$, $CCl_2CCl_2H$, $CBr_2CBr_2H$, $CCl_2CCl_2F$, $CBr_2CBr_2F$ usw.

Eine bevorzugte Gruppe von Mikrobiziden bilden Verbindungen im Umfang der Formel I, worin der Substituent $R_7$ für Trichlormethyl, Dichlorfluormethyl oder 1,1,2,2-Tetrachlorfluoräthyl steht. Eine andere wichtige Gruppe besteht aus denjenigen Verbindungen im Umfang der Formel I, worin der Substituent $R_7$ für 1,1,2,2-Tetrachloräthyl steht.

Unter Alkyl oder als Alkyl-Teil einer Alkoxygruppe sind je nach Definition der Kettenlänge folgende Gruppen zu verstehen: Methyl, Aethyl, n-Propyl, i-Propyl sowie die vier isomeren Butylreste.

Die Verbindungen der Formel I repräsentieren zwei Reihen von Wirkstoff-Typen, die dadurch gekennzeichnet sind, dass der Substituent $R_5$

a) eine α-Propionsäure(thio)estergruppe und

b) eine γ-Butyro(thio)lactongruppe darstellt.

Unter den Estern a) sind die -O-Methylester bevorzugt, und vor allem solche, in denen der Rest $R_6$ eine Methyl- oder Aethylgruppe darstellt.

Die Gruppe der unter a) genannten Ester-Reihe setzt sich aus zwei Untergruppen zusammen, in denen der Rest Ar

c) ein gegebenenfalls substituierter Naphthylrest oder

d) ein gegebenenfalls substituierter Phenyl-Rest ist.

Der hier und im folgenden verwendete Ausdruck "gegebenenfalls substituierter Naphthylrest" steht für den unter Formel I genannten 2-Methylnaphthylrest, in welchem $R_4$ wahlweise Wasserstoff oder Methyl ist.

Unter den Lactonen der obengenannten Reihe b) sind diejenigen bevorzugt, in denen X Sauerstoff darstellt, und vor allem solche, bei denen $R_6$ Methyl bedeutet.

Die Gruppe der unter b) genannten Lacton-Reihe setzt sich gleichfalls aus zwei Untergruppen zusammen, in denen der Rest Ar

e) ein gegebenenfalls substituierter Naphthylrest oder

f) ein gegebenenfalls substituierter Phenyl-Rest ist.

Unter den Verbindungen der Gruppen d) und f), in denen Ar einen gegebenenfalls substituierten Phenylrest bedeutet, stellen diejenigen eine bevorzugte Untergruppe dar, die in 2- und 6-Stellung anders als durch Wasserstoff substituiert sind.

Eine weitere Bevorzugung besteht darin, dass die 2-Position des Phenylrings durch eine Methylgruppe substituiert ist und die Reste $R_1$ bis $R_4$ zusammen nicht mehr als 6 Kohlenstoffatome enthalten.

Phenylacylalanine mit mikrobiziden Wirkungen sowie ihre Herstellung sind in der Literatur schon seit einiger Zeit bekannt. Beispielsweise seien folgende Veröffentlichungen genannt: DOS 2 741 437, 2 943 019, 2 947 295, 2 948 658 und 2 948 734.

Bekanntlich entfalten herkömmliche Phenylacylalanine ihre Aktivität fast ausschliesslich auf dem Pflanzenschutzgebiet und eignen sich hauptsächlich für die Bekämpfung von Pilzspezies aus der Familie der Oomycetes, beispielsweise von Phytophthora-, Pythium- oder Plasmopara-Erregern. Dagegen sind sie gegen andere Pilzarten, wie z.B. gegen Spezies aus der Familie der Fungi imperfecti nur unbefriedigend wirksam. Herkömmliche Phenylacylalanine eignen sich auch nicht zur Konservierung von verderblichen Waren, da sie im allgemeinen gegen die im Vorratsschutz massgebliche Mikro-Flora kaum aktiv sind.

Verbindungen der Formel I besitzen überraschenderweise nicht nur eine ausgeprägte Wirkung gegen die genannten Oomyceten und eine Reihe weiterer Pilzfamilien (Ascomyceten, Basidiomyceten), sondern eine zusätzliche Wirkung gegen wichtige Schadpilze aus der Familie der Fungi imperfecti, wie z.B. Cercospora, Piricularia und vor allem Botrytis. Botrytis spp. (B. cinerea, B. allii) stellen mit der Grauschimmelfäule an Reben, Erdbeeren, Aepfeln, Zwiebeln und anderen Obst- und Gemüsesorten einen bedeutenden wirtschaftlichen Schadfaktor dar. Darüberhinaus lassen sich Verbindungen der Formel I erfolgreich zum Schutz verderblicher Waren pflanzlicher oder tierischer Herkunft einsetzen. Sie bekämpfen Schimmelpilze wie Penicillium, Aspergillus, Rhizopus, Fusarium, Helminthosporium, Nigrospora und Alternaria sowie Bakterien wie Buttersäurebakterien und Hefen wie Candida.

Als Pflanzenschutzmittel besitzen die Verbindungen der Formel I ein für die praktischen Bedürfnisse sehr günstiges Wirkungsspektrum zum Schutze von Kulturpflanzen, ohne diese durch unerwünschte Nebenwirkungen nachteilig zu beeinflussen. Kulturpflanzen seien im Rahmen vorliegender Erfindung beispielsweise Getreide, Mais, Reis, Gemüse, Zuckerrüben, Soja, Erdnüsse, Obstbäume, Zierpflanzen, Reben, Hopfen, Gurkengewächse (Gurken, Kürbis, Melonen), Solanaceen, wie Kartoffeln, Tomaten und Tabak, sowie auch Bananen-, Kakao-, Avocado- und Naturkautschuk-Gewächse.

Mit den Wirkstoffen der Formel I können an Pflanzen oder Pflanzenteilen (Früchte, Blüten, Laubwerk, Stengel, Knollen, Wurzeln) dieser und verwandter Nutzkulturen die auftretenden Pilze eingedämmt oder vernichtet werden, wobei auch später zuwachsende Pflanzenteile von derartigen Pilzen verschont bleiben. Die Wirkstoffe sind gegen die den folgenden Klassen angehörenden phytopathogenen Pilze wirksam: Ascomycetes (z.B. Erysiphaceae, Fusarium, Helminthosporium); Basidiomycetes, wie vor allem Rostpilze (z.B. Puccinia, Tilletia); Fungi imperfecti (z.B. Moniliales u.a., Cercospora, Sclerotinia, Botrytis, Piricularia) und die der Klasse der Oomycetes angehörenden Phytophthora, Peronospora, Pseudoperonospora, Pythium oder Plasmopara. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Als Vorratsschutzmittel werden die Verbindungen der Formel I in unveränderter Form oder vorzugsweise zusammen mit den in der Formulierungstechnik üblichen Hilfsmitteln eingesetzt und werden z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B.

polymeren Stoffen in bekannter Weise verarbeitet. Die Anwendungsverfahren wie Versprühen, Vernebeln, Verstäuben, Verstreuen, Bestreichen oder Giessen und die Form des Mittels werden den angestrebten Zielen und den gegebenen Verhältnissen angepasst. Günstige Aufwandmengen liegen im allgemeinen bei 0,01 bis höchstens 2 kg Aktivsubstanz pro 100 kg zu schützendes Substrat; sie hängen jedoch ganz wesentlich von der Beschaffenheit (Grösse der Oberfläche, Konsistenz, Feuchtigkeitsgehalt) des Substrats und dessen Umgebungseinflüssen ab.

Unter Lager- und Vorratsgütern sollen im Rahmen vorliegender Erfindung pflanzliche und/oder tierische Naturstoffe und deren Weiterverarbeitungsprodukte verstanden werden, beispielsweise die nachfolgend aufgezählten und aus dem natürlichen Lebenszyklus herausgenommenen Pflanzen, deren Pflanzenteile (Stengel, Blätter, Knollen, Samen, Früchte, Körner) die im frisch geernten Zustand oder in weiterverarbeiteter Form vorliegen (vorgetrocknet, befeuchtet, zerkleinert, gemahlen, geröstet). Als Beispiele, die keinen, das Anwendungsgebiet limitierenden Charakter im Rahmen dieser Erfindung besitzen, seien folgende Ertragsgüter genannt: Getreide (wie Weizen, Gerste, Roggen, Hafer, Reis, Sorghum und Verwandte); Rüben (wie Möhren, Zucker- und Futterrüben); Kern-, Stein- und Beerenobst (wie Aepfel, Birnen, Pflaumen, Pfirsiche, Mandeln, Kirschen, Erd-, Him- und Brombeeren); Hülsenfrüchte (wie Bohnen, Linsen, Erbsen, Soja); Oelkulturen (wie Raps, Senf, Mohn, Oliven, Sonnenblumen, Kokos, Rizinus, Kakao, Erdnüsse); Gurkengewächse (wie Kürbis, Gurken, Melonen); Fasergewächse (wie Baumwolle, Flachs, Hanf, Jute, Nessel); Citrusfrüchte; Gemüsesorten (wie Spinat, Salat, Spargel, Kohlarten, Zwiebeln, Tomaten, Kartoffeln, Paprika): Loorbeergewächse (wie Avocado, Cinnamonum, Kampfer) oder Mais, Tabak, Nüsse, Kaffee, Zuckerrohr, Tee, Weintrauben, Kastanien, Hopfen, Bananen, Gras und Heu.

Als Naturprodukte tierischer Herkunft seien insbesondere getrocknete Fleisch- und Fischverarbeitungsprodukte wie Trockenfleisch, Trockenfisch, Fleischkonzentrate, Knochenmehl, Fischmehl und Tiertrockenfutter genannt.

Das behandelte Vorratsgut wird durch Behandlung mit Verbindungen der Formel I nachhaltig vor dem Befall durch Schimmelpilze und andere unerwünschte Mikroorganismen geschützt. Dadurch wird die Bildung toxischer und zum Teil karzinogener Schimmelpilzgifte (Aflatoxine und Ochratoxine) unterbunden, das Gut vor dem Verderben bewahrt und dessen Qualität für längere Zeit aufrechterhalten. Das erfindungsgemässe Verfahren kann auf alle trockenen und feuchten Vorrats- und Lagergüter angewendet werden, die gegen Mikroorganismen, wie Hefen, Bakterien und insbesondere Schimmelpilze anfällig sind.

Ein bevorzugtes Verfahren zum Aufbringen des Wirkstoffes besteht in einem Besprühen oder Benetzen des Substrats mit einer flüssigen Aufbereitung oder im Vermischen des Substrats mit einer festen Aufbereitung der Aktivsubstanz. Das beschriebene Verfahren ist ein Teil der vorliegenden Erfindung.

Die Verbindungen der Formel I lassen sich erfindungsgemäss nach folgendem Verfahren herstellen.

Nach einer ersten Variante werden die Verbindungen der Formel I hergestellt, indem man ein Sulfamoyloxyacetamid der Formel II

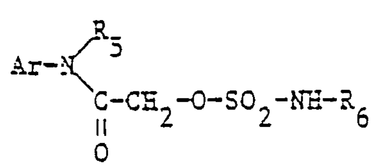

(II)

in Gegenwart einer Base mit einem Sulfenylchlorid der Formel III

$R_7$-S-Cl (III)

umsetzt.

Nach einer weiteren Variante kann man die Verbindungen der Formel I auch herstellen, indem man ein Amin der Formel IV

Ar-NH-$R_5$ (IV)

gegebenenfalls in Gegenwart einer Base mit einem Hydroxyessigsäure-Derivat der Formel V

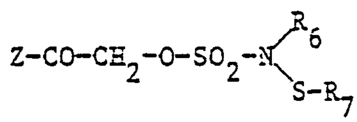

(V)

umsetzt, wobei in den Formeln II bis V Ar und $R_5$ bis $R_7$ die unter Formel I angegebene Bedeutung haben und Z ein aktivierender Substituent ist, vorzugsweise ein Halogenatom, im besonderen Chlor.

Die Umsetzungen können in gegenüber den Reaktionspartnern inerten organischen Lösungsmitteln vorgenommen werden. Als Beispiele seien genannt: Kohlenwasserstoffe wie Benzol, Toluol, Xylol oder Cyclohexan; Ester wie Aethylacetat; Alkohole wie Methanol, Aethanol oder Isopropanol sowie entsprechende Alkohol-Wasser-Gemische; halogenierte Kohlenwasserstoffe wie Methylenchlorid oder Chloroform; schliesslich Aether wie Diäthyläther, Tetrahydrofuran oder Dioxan.

Die Reaktionen können bei Temperaturen zwischen -20°C und 100°C ausgeführt werden. Der bevorzugte Temperaturbereich liegt zwischen 0°C und 30°C.

Als Basen können beispielsweise folgende Substanzen verwendet werden: Trialkylamine wie Triäthylamin; Hydroxyde wie Natrium- oder Kaliumhydroxyd; Carbonate wie Natrium- oder Kaliumcarbonat; Hydrogencarbonate wie Natriumhydrogencarbonat oder Hydride wie Natrium- oder Calciumhydrid.

Ein Teil der Ausgangsstoffe der Formel II sind in der DOS 2 741 437 beschrieben, die übrigen können in analoger Weise leicht hergestellt werden.

Ausgangsmaterialien der Formeln III, IV und V sind bekannt oder können in üblicher, literaturbekannter Weise erhalten werden.

Die Verbindungen der Formel I besitzen in $R_5$ ein asymmetrisches Kohlenstoffatom und können auf übliche Art in optische Antipoden gespalten werden. Die Antipoden der Formel I besitzen unterschiedliche mikrobizide Wirkungen.

Je nach Substitution können weitere asymmetrische Kohlenstoffatome im Molekül vorhanden sein.

Unabhängig von der genannten optischen Isomerie wird in der Regel eine Atropisomerie um die Aryl-N Achse beobachtet, die auf die sterische Hinderung der freien Drehbarkeit durch die ortho-Substitution im Arylkern zurückzuführen ist.

Sofern keine gezielte Synthese zur Isolierung reiner Isomerer durchgeführt wird, fällt normalerweise ein Produkt der Formel I als Gemisch dieser möglichen Isomeren an.

Die Verbindungen der Formel I können für sich allein oder zusammen mit geeigneten Trägern und/oder anderen Zuschlagstoffen verwendet werden. Geeignete Träger- und Zuschlagstoffe können fest oder flüssig sein und entsprechen den in der Formulierungstechnik üblichen Stoffen wie z.B. natürlichen oder regenerierten mineralischen Stoffen, Lösungs-, Dispergier-, Netz-, Haft-, Verdickungs-, Binde- oder Düngemitteln und hängen im Wesentlichen vom Verwendungszweck ab. So kommen auf dem Vorratsschutzgebiet nur solche Zuschlagstoffe in Frage, die ernährungsphysiologisch unbedenklich sind.

Der Gehalt an Wirkstoff in handelsfähigen Mitteln liegt zwischen 0,1 bis 90%.

Zur Applikation können die Verbindungen der Formel I in den folgenden Aufarbeitungsformen vorliegen (wobei die Gewichts-Prozentangaben in Klammern vorteilhafte Mengen an Wirkstoff darstellen):

Feste Aufarbeitungsformen:
Stäubemittel und Streumittel (bis zu 10%), Granulate, Umhüllungsgranulate, Imprägnierungsgranulate und Homogengranulate, Pellets (Körner) (1 bis 80%);

Flüssige Aufarbeitungsformen:
a) in Wasser dispergierbare Wirkstoffkonzentrate: Spritzpulver (wettable powders) und Pasten (25-90% in der Handelspackung, 0,01 bis 15% in gebrauchsfertiger Lösung); Emulsions- und Lösungskonzentrate (10 bis 50%; 0,01 bis 15% in gebrauchsfertiger Lösung);
b) Lösungen (0,1 bis 20%); Aerosole.

Solche Mittel gehören ebenfalls zur Erfindung.

Den beschriebenen erfindungsgemässen Mitteln lassen sich zur Verbreiterung des Wirkungsspektrums andere biozide Wirkstoffe oder Mittel beimischen. So können die neuen Mittel ausser den genannten Verbindungen der allgemeinen Formel I z.B. Insektizide, Herbizide, Bakteriostatika oder Nematizide weitere Fungizide, oder weitere Konservierungshilfsstoffe enthalten.

Die nachfolgenden Beispiele dienen zur näheren Erläuterung der Erfindung, ohne dieselbe einzuschränken. Die Temperaturen sind in Calsiusgraden angegeben. Prozente und Teile beziehen sich auf Gewicht. Sofern nicht anders vermerkt, ist bei der Nennung eines Wirkstoffs der Formel I stets das racemische Gemisch gemeint.

Herstellungsbeispiele

Beispiel 1: Herstellung von N-(1-Methoxycarbonyläthyl)-N-[(N'-dichlorfluormethylthio-N'-methyl-sulfamoyloxy)-acetyl]-2,6-dimethylanilin der Formel

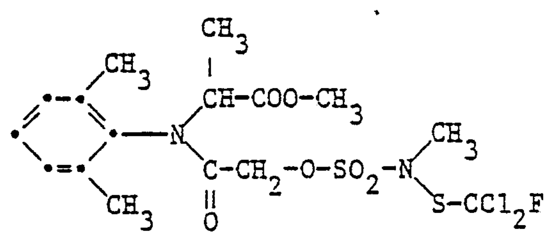

Zur auf 5° bis 10°C gekühlten Lösung von 17,9 g N-(1'-Methoxycarbonyläthyl)-N-(methylsulfamoyloxy-acetyl)-2,6-dimethylanilin in 150 ml Aethylacetat lässt man nacheinander 10,2 g Dichlorfluormethansulfenylchlorid und 6,6 g Triäthylamin zutropfen. Nachdem die Reaktionsmischung unter Erwärmung auf 20°C für 15 Stunden gerührt worden ist, wird der entstandene Niederschlag abfiltriert, das Filtrat mit 5%iger Natriumhydroxid-, 5%iger Salzsäurelösung und Wasser gewaschen, über Natriumsulfat getrocknet und eingedampft. Man erhält 18 g N-(1'-Methoxycarbonyläthyl)-N-[N'-dichlorfluormethylthio-N'-methyl-sulfamoyloxy)-acetyl]-2,6-dimethylanilin als braunes Oel, $n_D^{42}$= 1,5139 (Verb. 1.1.).

Beispiel 2: Herstellung von 3-[N-(N'-Trichlormethylthio-N'-methylsulfamoyloxy-acetyl)-N-(2,3,6-trimethylphenyl)-amino]-tetrahydro-2-furanon der Formel

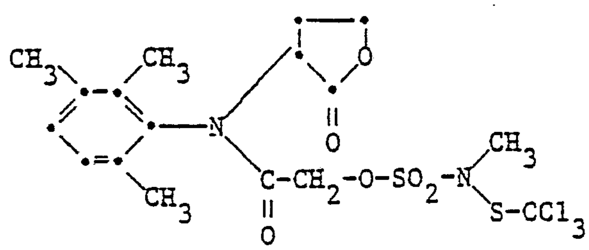

Zur auf 0° bis 5°C gekühlten Lösung von 10 g 3-[N-(Methyl-sulfamoyloxy-acetyl)-N-(2,3,6-trimethylphenyl)-amino]-tetrahydro-2-furanon in 150 ml Aethylacetat lässt man nacheinander 6 g Trichlormethylsulfenylchlorid und 3,5 g Triäthylamin zutropfen. Nachdem die Reaktionsmischung unter Erwärmung auf 20°C für 15 Stunden gerührt worden ist, werden 200 ml 5%ige Natriumhydroxidlösung zugesetzt, die organische Phase abgetrennt und mit Wasser neutral gewaschen, über Natriumsulfat getrocknet und eingeengt. Das ölige Rohprodukt wird durch Chromatographie an 500 g Kieselgel mit Chloroform-Aether-Gemisch (1:1) gereinigt. Man erhält 9,5 g 3-[N-(N'-Trichlormethylthio-N'-methyl-sulfamoyloxy-acetyl)-N-(2,3,6-trimethylphenyl)-amino]-tetrahydro-2-furanon vom Schmelzpunkt 53-58°C (Verb. 2.10.).

Die nach den Herstellungsbeispielen erhaltenen und analog hergestellten Verbindungen sind in den anschliessenden Tabellen 1 bis 3 aufgeführt:

Tabelle 1

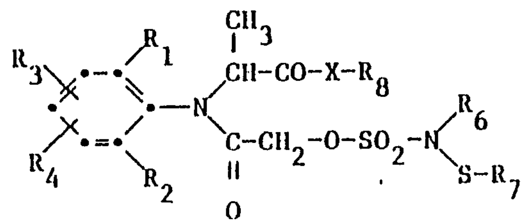

| Nr. | $R_1$ | $R_2$ | $R_3$ | $R_4$ | $R_6$ | $R_7$ | $R_8$ | X | Physik. Daten |
|---|---|---|---|---|---|---|---|---|---|
| 1.1. | $CH_3$ | $CH_3$ | H | H | $CH_3$ | $-CCl_2F$ | $CH_3$ | O | $n_D^{42}$ = 1,5139 |
| 1.2. | $CH_3$ | $CH_3$ | H | H | $CH_3$ | $-CCl_3$ | $CH_3$ | O | $n_D^{49}$ = 1,5200 |
| 1.3. | $CH_3$ | $CH_3$ | H | H | $CH_3$ | $-CCl_2F$ | $CH_3$ | S | |
| 1.4. | $CH_3$ | $CH_3$ | H | H | $CH_3$ | $-CCl_2F$ | $C_2H_5$ | O | |
| 1.5. | $CH_3$ | $CH_3$ | H | H | $CH_3$ | $-CCl_3$ | 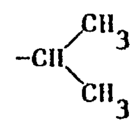 | O | |
| 1.6. | $CH_3$ | $CH_3$ | H | H | $CH_3$ | $-CCl_3$ | $-CH_2CH_2Cl$ | O | |
| 1.7. | $CH_3$ | $CH_3$ | H | H | $CH_3$ | $-CCl_2F$ | $-CH_2CH_2OCH_3$ | O | |
| 1.8. | $CH_3$ | $CH_3$ | H | H | $C_2H_5$ | $-CCl_2F$ | $CH_3$ | O | $n_D^{42}$ = 1,5115 |
| 1.9. | $CH_3$ | $CH_3$ | H | H | $C_2H_5$ | $-CCl_3$ | $CH_3$ | O | $n_D^{49}$ = 1,5219 |
| 1.10. | $CH_3$ | $CH_3$ | 3-$CH_3$ | H | $CH_3$ | $-CCl_2F$ | $CH_3$ | O | $n_D^{42}$ = 1,5141 |

| Nr. | $R_1$ | $R_2$ | $R_3$ | $R_4$ | $R_6$ | $R_7$ | $R_8$ | X | Physik. Daten |
|---|---|---|---|---|---|---|---|---|---|
| 1.11. | $CH_3$ | $CH_3$ | $3\text{-}CH_3$ | $5\text{-}CH_3$ | $CH_3$ | $-CCl_2F$ | $CH_3$ | O | |
| 1.12. | $CH_3$ | $CH_3$ | 3-Cl | H | $CH_3$ | $-CCl_3$ | $CH_3$ | O | |
| 1.13. | $CH_3$ | Cl | H | H | $C_2H_5$ | $-CCl_3$ | $CH_3$ | O | |
| 1.14. | $CH_3$ | $OCH_3$ | H | H | $CH_3$ | $-CCl_2F$ | $CH_3$ | O | $n_D^{49} = 1{,}5015$ |
| 1.15. | $CH_3$ | $NO_2$ | H | H | $CH_3$ | $-CCl_3$ | $CH_3$ | O | |
| 1.16. | $CH_3$ | $CH_3$ | $3\text{-}NO_2$ | H | $CH_3$ | $-CCl_3$ | $CH_3$ | O | |
| 1.17. | $CH_3$ | $CF_3$ | H | H | $C_2H_5$ | $-CCl_2F$ | $CH_3$ | O | |
| 1.18. | $CH_3$ | $NH_2$ | H | H | $CH_3$ | $-CCl_3$ | $CH_3$ | O | |
| 1.19. | $CH_3$ | $CH_3$ | 3-CN | H | $CH_3$ | $-CCl_2F$ | 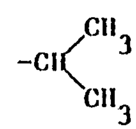 | O | |
| 1.20. | $CH_3$ | $CH_3$ | $3\text{-}CH_2OCH_3$ | H | $CH_3$ | $-CCl_3$ | $C_2H_5$ | S | |
| 1.21. | $CH_3$ | $CH_3$ | H | H | $C_3H_7(n)$ | $-CCl_2F$ | $CH_3$ | O | $n_D^{49} = 1{,}5022$ |
| 1.22. | $CH_3$ | $CH_3$ | H | H | $C_4H_9(n)$ | $-CCl_3$ | $CH_3$ | O | $n_D^{49} = 1{,}5252$ |
| 1.23. | Cl | $OCH_3$ | H | H | $CH_3$ | $-CCl_2F$ | $CH_3$ | O | |

| Nr. | $R_1$ | $R_2$ | $R_3$ | $R_4$ | $R_6$ | $R_7$ | $R_8$ | X | Physik. Daten |
|---|---|---|---|---|---|---|---|---|---|
| 1.24. | $CH_3$ | $CH_3$ | H | H | $CH_3$ | $-CCl_2CCl_2F$ | $CH_3$ | O | |
| 1.25. | $CH_3$ | $CH_3$ | 3-$CH_3$ | H | $CH_3$ | $-CCl_2CCl_2F$ | $CH_3$ | O | |
| 1.26. | $CH_3$ | $CH_3$ | H | H | $C_2H_5$ | $-CCl_2CCl_2F$ | $CH_3$ | O | |
| 1.27. | $CH_3$ | $CH_3$ | H | H | $C_3H_7$(n) | $-CCl_3$ | $CH_3$ | O | Oel |
| 1.28. | $CH_3$ | $CH_3$ | H | H | $C_4H_9$(n) | $-CCl_2F$ | $CH_3$ | O | $n_D^{49}$= 1,5048 |
| 1.29. | $CH_3$ | $CH_3$ | 3-$CH_3$ | H | $CH_3$ | $-CCl_3$ | $CH_3$ | O | Oel |
| 1.30. | $CH_3$ | $CH_3$ | 3-$CH_3$ | H | $C_2H_5$ | $-CCl_2F$ | $CH_3$ | O | $n_D^{42}$= 1,5161 |
| 1.31. | $CH_3$ | $CH_3$ | 3-$CH_3$ | H | $C_2H_5$ | $-CCl_3$ | $CH_3$ | O | $n_D^{49}$= 1,5258 |
| 1.32. | $CH_3$ | $CH_3$ | H | H | $CH_3$ | $-CCl_2CCl_2H$ | $CH_3$ | O | Smp. 102-108° |
| 1.33. | $CH_3$ | $CH_3$ | 3-$CH_3$ | H | $CH_3$ | $-CCl_2CCl_2H$ | $CH_3$ | O | Smp. 96-101° |
| 1.34. | Cl | $OC_2H_5$ | H | H | $CH_3$ | $-CCl_3$ | $CH_3$ | O | Oel |
| 1.35. | Cl | $OC_2H_5$ | H | H | $CH_3$ | $-CCl_2F$ | $CH_3$ | O | $n_D^{37}$= 1,4893 |
| 1.36. | $CH_3$ | $OCH_3$ | H | H | $CH_3$ | $-CCl_3$ | $CH_3$ | O | $n_D^{54}$= 1,3790 |

Tabelle 2

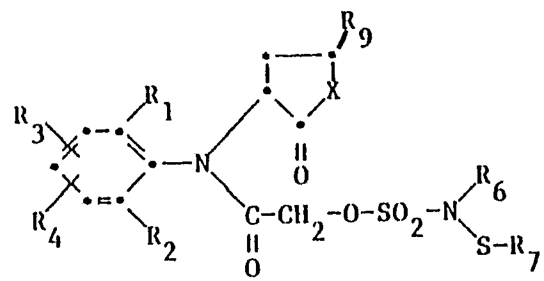

| Nr. | $R_1$ | $R_2$ | $R_3$ | $R_4$ | $R_6$ | $R_7$ | $R_9$ | X | Physik. Daten |
|---|---|---|---|---|---|---|---|---|---|
| 2.1. | $CH_3$ | $CH_3$ | H | H | $CH_3$ | $-CCl_2F$ | H | O | Smp. 121-125°C |
| 2.2. | $CH_3$ | $CH_3$ | H | H | $CH_3$ | $-CCl_3$ | H | O | Smp. 139-146°C |
| 2.3. | $CH_3$ | $CH_3$ | H | H | $CH_3$ | $-CCl_2F$ | H | O | Oel |
| 2.4. | $CH_3$ | $CH_3$ | H | H | $C_2H_5$ | $-CCl_3$ | H | O | |
| 2.5. | $CH_3$ | $CH_3$ | H | H | $C_3H_7$(n) | $-CCl_2F$ | H | O | |
| 2.6. | $CH_3$ | $CH_3$ | H | H | $CH_3$ | $-CCl_2F$ | $CH_3$ | O | |
| 2.7. | $CH_3$ | $CH_3$ | H | H | $CH_3$ | $-CCl_3$ | H | S | |
| 2.8. | $CH_3$ | $CH_3$ | 3-$CH_3$ | H | $CH_3$ | $-CCl_2F$ | H | O | Fliesspkt. 52-55°C |
| 2.9. | $CH_3$ | $CH_3$ | 3-$CH_3$ | H | $CH_3$ | $-CCl_3$ | H | O | Smp. 53-58°C |
| 2.10. | $CH_3$ | $CH_3$ | 3-$CH_3$ | H | $C_2H_5$ | $-CCl_3$ | H | O | $n_D^{50}$ = 1,5060 |

| Nr. | $R_1$ | $R_2$ | $R_3$ | $R_4$ | $R_6$ | $R_7$ | $R_9$ | X | Physik. Daten |
|---|---|---|---|---|---|---|---|---|---|
| 2.11. | $CH_3$ | $CH_3$ | $3-CH_3$ | H | $C_2H_5$ | $-CCl_2F$ | H | O | Smp. 77-84°C |
| 2.12. | $CH_3$ | $CH_3$ | $3-CH_3$ | $5-CH_3$ | $CH_3$ | $-CCl_2F$ | H | O | Smp. 98-101°C |
| 2.13. | $CH_3$ | $CH_3$ | $3-CH_3$ | $5-CH_3$ | $CH_3$ | $-CCl_3$ | H | O | Smp. 119-126°C |
| 2.14. | $CH_3$ | $CH_3$ | $3-CH_3$ | $5-CH_3$ | $C_2H_5$ | $-CCl_2F$ | H | O | Smp. 90-98°C |
| 2.15. | $CH_3$ | $CH_3$ | $3-CH_3$ | $5-CH_3$ | $C_2H_5$ | $-CCl_3$ | H | O | Smp. 118-122°C |
| 2.16. | $CH_3$ | $CH_3$ | 3-Cl | H | $CH_3$ | $-CCl_2F$ | H | O | |
| 2.17. | $CH_3$ | $CH_3$ | $3-CH_3$ | H | $CH_3$ | $-CCl_2F$ | H | S | |
| 2.18. | $CH_3$ | $NO_2$ | H | H | $CH_3$ | $-CCl_3$ | H | O | |
| 2.19. | $CH_3$ | $CH_3$ | $3-NO_2$ | H | $CH_3$ | $-CCl_3$ | H | O | |
| 2.20. | $CH_3$ | $CH_3$ | H | H | $C_2H_5$ | $-CCl_3$ | $CH_3$ | O | |
| 2.21. | $CH_3$ | $CH_3$ | H | H | $C_2H_5$ | $-CCl_2F$ | H | S | |
| 2.22. | $CH_3$ | $CH_3$ | H | H | $CH_3$ | $-CCl_2CCl_2F$ | H | O | Viscos |
| 2.23. | $CH_3$ | $CH_3$ | $3-CH_3$ | H | $C_2H_5$ | $-CCl_2CCl_2F$ | H | O | |
| 2.24. | $CH_3$ | $CH_3$ | H | H | $CH_3$ | $-CCl_2CCl_2H$ | H | O | Harz |

Tabelle 3

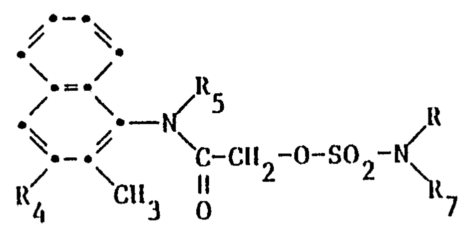

| Nr. | $R_4$ | $R_5$ | $R_6$ | $R_7$ | Physikalische Daten |
|---|---|---|---|---|---|
| 3.1. | H | $-CH(CH_3)COOCH_3$ | $CH_3$ | $-CCl_2F$ | $n_D^{42} = 1,5420$ |
| 3.2. | H | $-CH(CH_3)COOCH_3$ | $CH_3$ | $-CCl_3$ | $n_D^{49} = 1,5559$ |
| 3.3. | $CH_3$ | $-CH(CH_3)COOCH_3$ | $CH_3$ | $-CCl_2F$ | Harz |
| 3.4. | $CH_3$ | $-CH(CH_3)COOCH_3$ | $CH_3$ | $-CCl_3$ | viscos |
| 3.5. | H | $-CH(CH_3)COOCH_3$ | $C_2H_5$ | $-CCl_2F$ | $n_D^{49} = 1,5375$ |
| 3.6. | H | $-CH(CH_3)COOCH_3$ | $C_2H_5$ | $-CCl_3$ | $n_D^{49} = 1,5325$ |

| Nr. | $R_4$ | $R_5$ | $R_6$ | $R_7$ | Physikalische Daten |
|---|---|---|---|---|---|
| 3.7. | H | 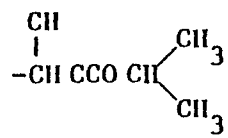 | $CH_3$ | $-CCl_3$ | |
| 3.8. | H | 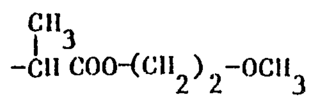 | $CH_3$ | $-CCl_2F$ | |
| 3.9. | H | 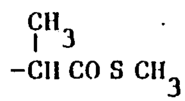 | $CH_3$ | $-CCl_3$ | |
| 3.10. | H | 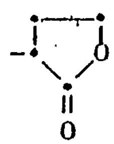 | $CH_3$ | $-CCl_2$ | Smp. 62-65°C |
| 3.11. | $CH_3$ | 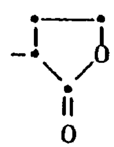 | $CH_3$ | $-CCl_2F$ | Smp. 110-112°C |
| 3.12. | H | 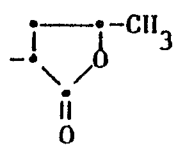 | $CH_3$ | $-CCl_2F$ | |

| Nr. | $R_4$ | $R_5$ | $R_6$ | $R_7$ | Physikalische Daten |
|---|---|---|---|---|---|
| 3.13. | H | 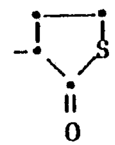 | $CH_3$ | $-CCl_2F$ | viscos |
| 3.14. | H | 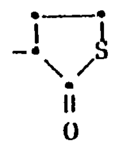 | $CH_3$ | $-CCl_3$ | |
| 3.15. | H | $CH_3$<br>\|<br>$-CH\ COO\ CH_3$ | $C_3H_7(n)$ | $-CCl_2F$ | Oel |
| 3.16. | H | $CH_3$<br>\|<br>$-CH\ COO\ CH_3$ | $C_4H_9(n)$ | $-CCl_3$ | |
| 3.17. | H | $CH_3$<br>\|<br>$-CH\ COO\ CH_3$ | $CH_3$ | $-CCl_2CCl_2F$ | Harz |
| 3.18. | H | 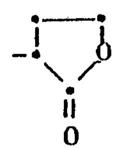 | $CH_3$ | $-CCl_2CCl_2F$ | |
| 3.19. | H | 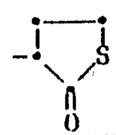 | $CH_3$ | $-CCl_2CCl_2F$ | |

| Nr. | $R_4$ | $R_5$ | $R_6$ | $R_7$ | Physikalische Daten |
|---|---|---|---|---|---|
| 3.20. | H | 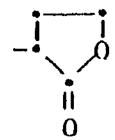 | $-C_3H_7$ (n) | $-CCl_2F$ | |
| 3.21. | H | 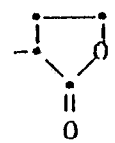 | $-C_3H_7$(n) | $-CCl_3$ | |
| 3.22 | H | 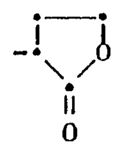 | $-C_4H_9$(n) | $-CCl_2F$ | Fliesspkt. 60°C |
| 3.23. | H | 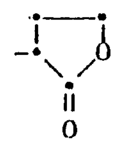 | $-C_4H_9$(n) | $-CCl_3$ | $n_D^{50}$= 1,5320 |
| 3.24. | H | 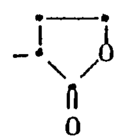 | $-C_2H_5$ | $-CCl_2F$ | Smp. 66-70°C |
| 3.25. | H | 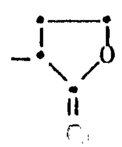 | $-C_2H_5$ | $-CCl_3$ | Fliesspkt. 50°C |

| Nr. | $R_4$ | $R_5$ | $R_6$ | $R_7$ | Physikalische Daten |
|---|---|---|---|---|---|
| 3.26. | H | 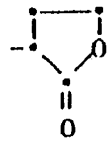 | $-CH_3$ | $-CCl_3$ | Smp. 60-70°C |
| 3.27. | $CH_3$ | 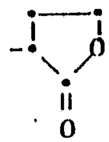 | $-CH_3$ | $-CCl_3$ | Smp. 76-84°C |
| 3.28. | $CH_3$ | 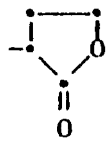 | $-C_2H_5$ | $-CCl_2F$ | Smp. 102-103°C |
| 3.29. | $CH_3$ | 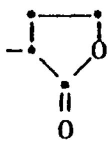 | $-C_2H_5$ | $-CCl_3$ | Smp. 123-125°C |
| 3.30. | $CH_3$ | $CH_3$<br>$-CH\ COO\ CH_3$ | $-C_2H_5$ | $-CCl_2F$ | Harz |
| 3.31. | $CH_3$ | $CH_3$<br>$-CH\ COO\ CH_3$ | $-C_2H_5$ | $-CCl_3$ | Harz |
| 3.32. | $CH_3$ | $CH_3$<br>$-CHCOOCH_3$ | $-CH_3$ | $-CCl_2CCl_2H$ | Oel |
| 3.33. | H | 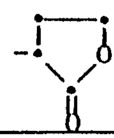 | $-CH_3$ | $-CCl_2CCl_2H$ | viscoses Oel |

## Formulierungsbeispiele

### Beispiel 1: Stäubemittel

Zur Herstellung eines a) 5%igen und b) 2%igen Stäubemittels werden die folgenden Stoffe verwendet:

a) 5 Teile Wirkstoff 1.1 bis 3.33
95 Teile Talkum;

b) 2 Teile Wirkstoff,
1 Teil hochdisperse Kieselsäure,
97 Teile Talkum;

Die Wirkstoffe werden mit den Trägerstoffen vermischt und vermahlen und können in dieser Form zur Anwendung verstäubt werden.

### Beispiel 2: Granulat

Zur Herstellung eines 5%igen Granulates werden die folgenden Stoffe verwendet:

5 Teile Wirkstoff 1.1 bis 3.33
0,25 Teile epoxidiertes Pflanzenöl,
0,25 Teile Cetylpolyglykoläther,
3,50 Teile Polyäthylenglykol,
91 Teile Kaolin (Korngrösse 0,3-0,8 mm).

Die Aktivsubstanz wird mit epoxidiertem Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht, und anschliessend wird das Aceton im Vakuum verdampft. Ein derartiges Mikrogranulat wird vorteilhaft zur Bekämpfung von Bodenpilzen verwendet.

Beispiel 3: Spritzpulver

Zur Herstellung eines a) 70%igen b) 40%igen c) und d) 25%igen e) 10%igen Spritzpulvers werden folgende Bestandteile verwendet:

a) 70 Teile Wirkstoff 1.1 bis 3.33
5 Teile Natriumdibutylnaphthylsulfonat,
3 Teile Naphthalinsulfonsäuren-Phenolsulfonsäuren-Formaldehyd-Kondensat 3:2:1,
10 Teile Kaolin,
12 Teile Kreide;

b) 40 Teile Wirkstoff 1.1 bis 3.33
5 Teile Ligninsulfonsäure-Natriumsalz,
1 Teil Dibutylnaphthalinsulfonsäure-Natriumsalz,
54 Teile Kieselsäure;

c) 25 Teile Wirkstoff 1.1 bis 3.33
4,5 Teile Calcium-Ligninsulfonat,
1,9 Teile Kreide/Hydroxyäthylcellulose-Gemisch (1:1),
1,5 Teile Natrium-dibutyl-naphthalinsulfonat,
19,5 Teile Kieselsäure,
19,5 Teile Kreide,
28,1 Teile Kaolin;

d) 25 Teile Wirkstoff 1.1 bis 3.33
2,5 Teile Isooctylphenoxy-polyoxyäthylen-äthanol,
1,7 Teile Kreide/Hydroxyäthylcellulose-Gemisch (1:1),
8,3 Teile Natriumaluminiumsilikat,
16,5 Teile Kieselgur,
46 Teile Kaolin;

e) 10 Teile Wirkstoff 1.1 bis 3.33
3 Teile Gemisch der Natriumsalze von gesättigten Fettalkoholsulfaten,
5 Teile Naphthalinsulfonsäure-Formaldehyd-Kondensat,
82 Teile Kaolin;

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation verwenden lassen.

Beispiel 4: Emulgierbare Konzentrate

Zur Herstellung eines 25%igen emulgierbaren Konzentrates werden folgende Stoffe verwendet:

25 Teile Wirkstoff 1.1 bis 3.33
2,5 Teile epoxydiertes Pflanzenöl,
10 Teile eines Alkylarylsulfonat/Fettalkoholpolyglykoläther-Gemisches,
5 Teile Dimethylformamid,
57,5 Teile Xylol.

Aus solchen Konzentraten können durch Verdünnen mit Wasser Emulsionen der gewünschten Anwendungskonzentration hergestellt werden, die besonders zur Blattapplikation geeignet sind.

## Biologische Beispiele

Beispiel 1: Wirkung gegen Phytophthora auf Tomatenpflanzen

a) Residual-protektive Wirkung: Tomatenpflanzen wurden nach 3-wöchiger Anzucht mit einer aus Spritzpulver des Wirkstoffes hergestellten Spritzbrühe (0,06% Aktivsubstanz) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100% relativer Luftfeuchtigkeit und 20°C.

b) Residual-kurative Wirkung: Tomatenpflanzen wurden nach 3-wöchiger Anzucht mit einer Sporangiensuspension des Pilzes infiziert. Nach einer Inkubation von 22 Stunden in einer Feuchtkammer bei 90-100% relativer Luftfeuchtigkeit und 20°C wurden die infizierten Pflanzen getrocknet und mit einer aus Spritzpulver des Wirkstoffes hergestellten Spritzbrühe (0,06% Aktivsubstanz) besprüht. Nach dem Antrocknen des Spritzbelages wurden die behandelten Pflanzen wieder in die Feuchtkammer gebracht. Die Beurteilung des Pilzbefalls erfolgte 5 Tage nach der Infektion.

c) Systemische Wirkung: Zu Tomatenpflanzen wurde nach 3-wöchiger Anzucht eine aus Spritzpulver des Wirkstoffes hergestellte Spritzbrühe gegossen (0,006% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100% relativer Luftfeuchtigkeit und 20°C.

In obigen Versuchen a, b und c reduzierten die Verbindungen 1.1, 1.2, 1.8, 1.9, 1.10, 1.27, 1.29, 1.30-1.33, 2.1, 2.2, 2.8, 2.9, 2.10, 2.11, 2.12, 2.13, 2.22, 2.27, 3.1, 3.10, 3.23, 3.24 und 3.26 den Phytophthora-Befall auf weniger als 10%, die Verbindungen Nr. 1.1, 1.2, 1,27, 2.27 und 3.24 verhinderten den Befall vollständig.

Beispiel 2: Wirkung gegen Plasmopara viticola auf Reben

Im 4-5 Blattstadium wurden Rebensämlinge mit einer aus Spritzpulver des Wirkstoffes hergestellten Spritzbrühe (0,06% Aktivsubstanz) besprüht. Nach 24 Stunden wurden die behandelten Pflanzen mit einer Sporangiensuspension des Pilzes infiziert. Nach einer Inkubation während 6 Tagen bei 95-100% relativer Luftfeuchtigkeit und 20°C wurde der Pilzbefall beurteilt.

Dabei zeigten unter anderen die Verbindungen Nr. 1.1, 1.2, 1.8, 1.9, 1.10, 1.27, 1.29, 1.30, 1.31, 2.1, 2.2, 2.8, 2.9, 2.10, 2.11, 2.12, 2.13, 2.22, 2.27, 3.1, 3.10, 3.23, 3.24 und 3.26 eine sehr gute fungizide Wirkung, sie reduzierten den Pilzbefall auf 0 bis 10%.

Beispiel 3: Wirkung gegen Pythium debaryanum auf Rüben

Wirkung nach Bodenapplikation. Der Pilz wurde auf Karottenschnitzel-Nährlösung kultiviert und einer Erde-Sand-Mischung beigegeben. Die so infizierte Erde wurde in Blumentöpfe abgefüllt und mit Zuckerrübensamen besät. Gleich nach der Aussaat wurden die als Spritzpulver formulierten Versuchspräparate als wässrige Suspensionen über die Erde gegossen (20 ppm Wirkstoff bezogen auf das Erdvolumen). Die Töpfe wurden darauf während 2-3 Wochen im Gewächshaus bei ca. 20°C aufgestellt. Die Erde wurde dabei durch leichtes Ueberbrausen stets gleichmässig feucht gehalten.

Bei der Auswertung der Tests wurde der Auflauf der Zuckerrübenpflanzen sowie der Anteil gesunder und kranker Pflanzen bestimmt.

Wirkung nach Beizapplikation. Der Pilz wurde auf Karottenschnitzel-Nährlösung kultiviert und einer Erde-Sand-Mischung beigegeben. Die so infizierte Erde wurde in Erdschalen abgefüllt und mit Zuckerrübensamen besät, die mit den als Beizpulver formulierten Versuchspräparaten gebeizt worden waren (0,06% Wirkstoff).

Die besäten Töpfe wurden während 2-3 Wochen im Gewächshaus bei ca. 20°C aufgestellt. Die Erde wurde dabei durch leichtes Ueberbrausen stets gleichmässig feucht gehalten. Bei der Auswertung wurde der Auflauf der Zuckerrübenpflanzen bestimmt.

Unter anderen zeigten die Verbindungen Nr. 1.1, 1.2, 1.8, 1.9, 1.27, 1.29, 1.30-1.33, 2.1, 2.2, 2.9, 2.10, 2.11, 2.22, 2.27, 3.1, 3.23, 3.24 und 3.26 eine sehr gute Wirkung gegen Pythium. Sowohl nach der Boden- als auch nach der Beizapplikation liefen mehr als 90% der Pflänzchen auf und zeigten ein gesundes Aussehen.

Beispiel 4: Wirkung gegen Cercorpora arachidicola auf Erdnusspflanzen

10-15 cm hohe Erdnusspflanzen wurden mit einer aus Spritzpulver der Wirksubstanz hergestellten Spritzbrühe (0,02% Aktivsubstanz) besprüht und 48 Stunden später mit einer Konidiensuspension des Pilzes infiziert. Die infizierten Pflanzen wurden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgte 12 Tage nach der Infektion basierend auf Anzahl und Grösse der auftretenden Flecken.

Durch Behandlung mit einer der Verbindungen Nr. 1.29, 2.9, 3.2, 3.28 und 3.29 wurde der Cercosporabefall auf weniger als 10% reduziert.

Beispiel 5: Wirkung gegen Piricularia oryzae auf Reis

Reispflanzen wurden nach zweiwöchiger Anzucht mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach 5 Tagen Inkubation bei 95-100% relativer Luftfeuchtigkeit und 24°C wurde der Pilzbefall beurteilt.

Durch Behandlung mit den Verbindungen Nr. 1.1 oder 1.2 wurde der Piriculariabefall auf weniger als 10% reduziert.

Beispiel 6: Wirkung gegen Botrytis cinerea auf Bohnenpflanzen

Ca. 10 cm hohe Bohnen-Pflanzen wurden mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach einer Inkubation der infizierten Pflanzen während 2-3 Tagen bei 95-100% relativer Luftfeuchtigkeit und 21°C erfolgte die Beurteilung des Pilzbefalls.

Beispiel 7: Wirkung gegen Botrytis cinerea auf Apfelfrüchten

Künstlich verletzte Aepfel wurden behandelt, indem eine aus Spritzpulver der Wirksubstanz hergestellte Spritzbrühe auf die Verletzungsstellen aufgetropft wurde. Die behandelten Früchte wurden anschliessend mit einer Sporensuspension von Botrytis cinerea inokuliert und während einer Woche bei hoher Luftfeuchtigkeit und ca. 20°C inkubiert. Bei der Auswertung wurden die angefaulten Verletzungsstellen gezählt und daraus die fungizide Wirkung der Testsubstanz abgeleitet.

In den vorstehenden Versuchen (Beispiele 6 und 7) bewirken folgende Verbindungen eine Reduktion des Krankheitsbefalls auf weniger als 20%: Verbindungen Nr. 1.1; 1.2; 1.9; 1.10; 1.29 bis 1.34; 1.36; 2.9; 2.10; 3.1 und 3.30.

Beispiel 8: Körnerschutz: ("Grainpreservative Test")

a) Kurzzeittest gegen Schimmelpilze an Feucht-Mais

Trockene, als Tierfutter vorgesehene Maiskörner (Portionen à 80 g) werden in verschliessbaren Plastikbechern mit Wirkstoffen der Formel I in Form einer wässrigen Suspension, Emulsion oder Lösung gut durchmischt. Die Substanzapplikation wird so bemessen, dass eine Konzentration von 0,06% AS bezüglich des Maistrockengewichts erreicht wird. Ein befeuchteter Papierlappen sorgt für eine feuchtigkeitsgesättigte Atmosphäre in den mit Mais gefüllten und anschliessend verschlossenen Bechern. Nach 2-3 Wochen Inkubation bei ca. 20°C entwickelt sich bei den nur mit Wasser behandelten Mais-Proben spontan eine Mischpopulation von Schimmelpilzen. Eine künstliche Infektion erübrigt sich. Das Ausmass der Pilzentwicklung nach 3 Wochen dient zur Bewertung der Wirksamkeit der Verbindungen Nr. 1.1 bis 3.33.

b) Langzeittest gegen Schimmelpilze an Feucht-Mais

I. Die Mais-Proben, die nach 3 Wochen keinen Pilzbefall aufweisen, werden während zwei weiteren Monaten inkubiert. Nach jedem Monat erfolgt eine visuelle Bonitur nach denselben Kriterien wie im a-Test.

II. Die Testdurchführung erfolgt grundsätzlich gleich wie unter a und bI, doch wird die zu prüfende Substanz bei 2000, 600 und 200 ppm AS (bezogen auf das Maistrockengewicht) während 6 Monaten geprüft.

So verhinderten die Verbindungen Nr. 1.1 bis 3.33 im a-Test bei einer Prüfkonzentration von 600 ppm AS den Schimmelpilzbefall fast vollständig (0-5%). Mit den Verbindungen Nr. 1.14, 1.34, 1.35 und 1.36 wurde ein gleich guter Konservierungsgrad bei einer Prüfkonzentration von 200 ppm in allen drei Tests a, bI und bII erreicht.

<u>Patentansprüche</u> (für alle benannten Länder ausser Oesterreich)

1. Verbindungen der Formel I

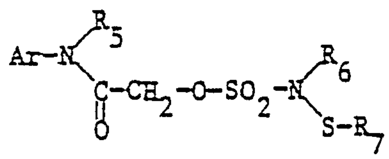

(I)

worin

Ar einen Rest der Formeln

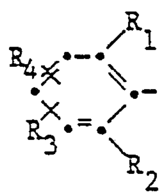

oder

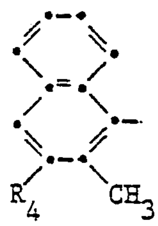

,

$R_1$ Wasserstoff, Chlor oder Methyl,

$R_2$ Wasserstoff, Chlor, Methyl, Nitro, Amino, Methylamino, Methoxy, Cyan oder Trifluormethyl,

$R_3$ Wasserstoff, Chlor, Methyl, Nitro, Amino, Cyan oder Alkoxymethyl mit nicht mehr als 5 Kohlenstoffatomen,

$R_4$ Wasserstoff oder Methyl,

$R_5$ einen Rest der Formel

$-CH(CH_3)-CO-X-R_8$ oder

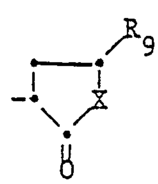

,

$R_6$ $C_1$-$C_4$-Alkyl,

$R_7$ Halo($C_1$-$C_2$)-alkyl),

$R_8$ gegebenenfalls durch Halogen oder Methoxy substituiertes $C_1$-$C_3$-Alkyl,

$R_9$ Wasserstoff oder Methyl und

X Sauerstoff oder Schwefel bedeuten.

2. Verbindungen der Formel I

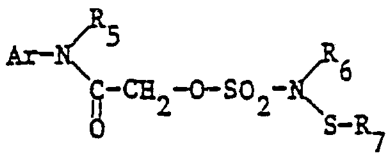 (I)

worin

Ar einen Rest der Formeln

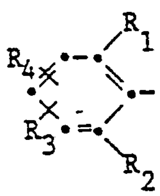 oder 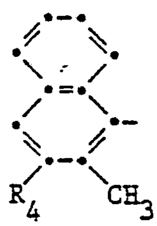 ,

$R_1$ Wasserstoff, Chlor oder Methyl,

$R_2$ Wasserstoff, Chlor, Methyl, Nitro, Amino, Methylamino, Methoxy, Cyan oder Trifluormethyl,

$R_3$ Wasserstoff, Chlor, Methyl, Nitro, Amino, Cyan oder Alkoxymethyl mit nicht mehr als 5 Kohlenstoffatomen,

$R_4$ Wasserstoff oder Methyl,

$R_5$ einen Rest der Formel

$-CH(CH_3)-CO-X-R_8$ oder 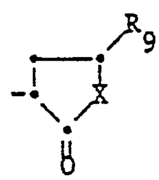 ,

$R_6$ $C_1$-$C_4$-Alkyl,

$R_7$ Trichlormethyl, Dichlorfluormethyl oder 1,1,2,2-Tetrachlorfluoräthyl,

$R_8$ gegebenenfalls durch Halogen oder Methoxy substituiertes $C_1$-$C_3$-Alkyl,

$R_9$ Wasserstoff oder Methyl und

X Sauerstoff oder Schwefel bedeuten.

3. Verbindungen gemäss Anspruch 2, dadurch gekennzeichnet, dass $R_5$ den Rest $-CH(CH_3)-CO-X-R_8$ darstellt.

4. Verbindungen gemäss Anspruch 3, dadurch gekennzeichnet, dass $R_8$ Methyl bedeutet.

5. Verbindungen gemäss Anspruch 4, dadurch gekennzeichnet, dass Ar den substituierten Naphthylrest darstellt.

6. Verbindungen gemäss Anspruch 4, dadurch gekennzeichnet, dass Ar den gegebenenfalls substituierten Phenylrest bedeutet.

7. Verbindungen gemäss Anspruch 6, dadurch gekennzeichnet, dass der Phenylkern in 2- und 6-Stellung anders als durch Wasserstoff substituiert ist.

8. Verbindungen gemäss Anspruch 7, dadurch gekennzeichnet, dass der Substituent in der 2-Stellung des Phenylkerns eine Methylgruppe ist, und dass die Substituenten $R_1$ bis $R_4$ zusammen nicht mehr als 6 Kohlenstoffatome enthalten.

9. Verbindungen gemäss Anspruch 2, dadurch gekennzeichnet, dass der Substituent $R_5$ den (Thio)Lactonrest bedeutet.

10. Verbindungen gemäss Anspruch 9, dadurch gekennzeichnet, dass $R_6$ Methyl oder Aethyl ist und im Lactonring X Sauerstoff bedeutet.

11. Verbindungen gemäss Anspruch 10, dadurch gekennzeichnet, dass Ar den gegebenenfalls substituierten Phenylrest bedeutet.

12. Verbindungen gemäss Anspruch 10, dadurch gekennzeichnet, dass Ar den substituierten Naphthylrest bedeutet.

13. Verbindungen gemäss Anspruch 11, dadurch gekennzeichnet, dass der Phenylkern in 2- und 6-Stellung anders als durch Wasserstoff substituiert ist.

14. Verbindungen gemäss Anspruch 13, dadurch gekennzeichnet, dass $R_1$ Methyl bedeutet und dass die Substituenten $R_1$ bis $R_4$ zusammen nicht mehr als 6 Kohlenstoffatome enthalten.

15. N-(1-Methoxycarbonyläthyl)-N-[(N'-methyl-N'-trichlormethylthio--sulfamoyloxy)-acetyl]-2,6-dimethylanilin gemäss Anspruch 2.

16. 3-[N-(N'-Dichlorfluormethylthio-N'-äthyl-sulfamoyloxy)-acetyl-N-(2-methylnaphthyl)-amino]-tetrahydrofuranon gemäss Anspruch 2.

17. Verbindungen gemäss Anspruch 1, worin $R_7$ den 1,1,2,2-Tetrachloräthylrest bedeutet, Ar eine substituierte Phenylgruppe darstellt, in der $R_1$ Methyl ist, und $R_5$ die Gruppe $-CH(CH_3)CO-X-CH_3$ bedeutet, in der X Sauerstoff oder Schwefel ist.

18. N-(1-Methoxycarbonyläthyl)-N-[(N'-methyl-N'-1,1,2,2-tetrachloräthylthiosulfamoyloxy)-acetyl]-2,6-dimethylanilin gemäss Anspruch 1.

19. Verfahren zur Herstellung der Verbindungen der Formel I, gemäss Anspruch 1, dadurch gekennzeichnet, dass man ein Sulfamoyloxy-acetamid der Formel II

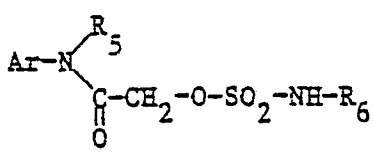

(II)

in Gegenwart einer Base mit einem Sulfenylchlorid der Formel III

$R_7-S-Cl$ (III)

worin Ar und $R_5$ bis $R_7$ die unter Formel I angegebene Bedeutung haben, umsetzt.

20. Verfahren zur Herstellung der Verbindungen der Formel I, gemäss Anspruch 1, dadurch gekennzeichnet, dass man ein Amin der Formel IV

$Ar-NH-R_5$ (IV)

gegebenenfalls in Gegenwart einer Base mit einem Hydroxyessigsäure-Derivat der Formel V

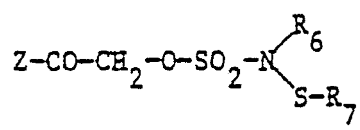

(V)

umsetzt, worin Ar und $R_5$ bis $R_7$ die unter Formel I angegebene Bedeutung haben und Z ein aktivierender Substituent ist.

21. Mikrobizides Mittel, dadurch gekennzeichnet, dass es neben Träger- und/oder anderen Zuschlagstoffen als wirksame Komponente mindestens eine Verbindung der Formel I, gemäss Anspruch 1, enthält.

22. Mittel gemäss Anspruch 21, dadurch gekennzeichnet, dass es als aktive Komponente eine Verbindung der Formel I gemäss einem der Ansprüche 2 bis 18 enthält.

23. Verwendung von Verbindungen der in Anspruch 1 definierten Formel I im Pflanzen- oder Vorratsschutz zur Bekämpfung schädlicher Pilze und Bakterien.

24. Verwendung von Verbindungen der im Anspruch 2 definierten Formel I zur Bekämpfung phytopathogener Pilze und Hefen.

Patentansprüche (für Oesterreich)

1. Mikrobizide Mittel enthaltend als Wirkstoff eine Verbindung der Formel I

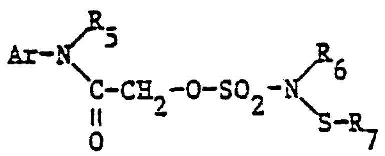 (I)

worin Ar einen Rest der Formeln

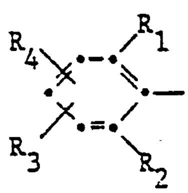 oder 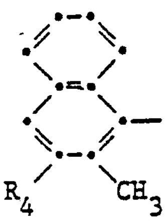 ,

$R_1$ Wasserstoff, Chlor oder Methyl,

$R_2$ Wasserstoff, Chlor, Methyl, Nitro, Amino, Methylamino, Methoxy, Cyan oder Trifluormethyl,

$R_3$ Wasserstoff, Chlor, Methyl, Nitro, Amino, Cyan oder Alkoxymethyl mit nicht mehr als 5 Kohlenstoffatomen,

$R_4$ Wasserstoff oder Methyl,

$R_5$ einen Rest der Formeln $-CH(CH_3)-CO-X-R_8$ oder 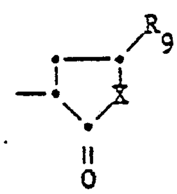 ,

$R_6$ $C_1$-$C_4$-Alkyl,

$R_7$ Halo($C_1$-$C_2$-alkyl),

$R_8$ gegebenenfalls durch Halogen oder Methoxy substituiertes $C_1$-$C_3$-Alkyl,

$R_9$ Wasserstoff oder Methyl und

X Sauerstoff oder Schwefel bedeuten,

zusammen mit geeigneten Trägerstoffen und gegebenenfalls weiteren applikationsfördernden Zusätzen.

2. Mikrobizide Mittel enthaltend als Wirkstoff eine Verbindung der Formel I

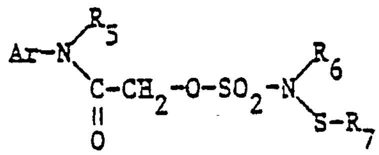

(I)

worin Ar einen Rest der Formeln

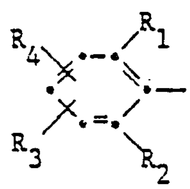

oder

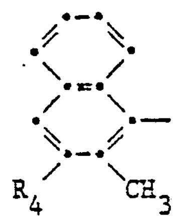

$R_1$ Wasserstoff, Chlor oder Methyl,

$R_2$ Wasserstoff, Chlor, Methyl, Nitro, Amino, Methylamino, Methoxy, Cyan oder Trifluormethyl,

$R_3$ Wasserstoff, Chlor, Methyl, Nitro, Amino, Cyan oder Alkoxymethyl mit nicht mehr als 5 Kohlenstoffatomen,

$R_4$ Wasserstoff oder Methyl,

$R_5$ einen Rest der Formeln $-CH(CH_3)-CO-X-R_8$ oder 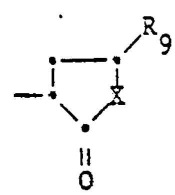 ,

$R_6$ $C_1$-$C_4$-Alkyl,

$R_7$ Trichlormethyl, Dichlormethyl oder 1,1,2,2-Tetrachlorfluoräthyl,

$R_8$ gegebenenfalls durch Halogen oder Methoxy substituiertes $C_1$-$C_3$-Alkyl,

$R_9$ Wasserstoff oder Methyl und

X Sauerstoff oder Schwefel bedeuten,

zusammen mit geeigneten Trägerstoffen und gegebenenfalls weiteren applikationsfördernden Zusätzen.

3. Mittel nach Anspruch 2 enthaltend als Wirkstoff eine Verbindung der Formel I, worin $R_5$ den Rest $-CH(CH_3)-CO-X-R_8$ darstellt.

4. Mittel nach Anspruch 3 enthaltend als Wirkstoff eine Verbindung der Formel I, worin $R_8$ Methyl bedeutet.

5. Mittel nach Anspruch 4 enthaltend als Wirkstoff eine Verbindung der Formel I, worin Ar den substituierten Naphthylrest darstellt.

6. Mittel nach Anspruch 4 enthaltend als Wirkstoff eine Verbindung der Formel I, worin Ar den gegebenenfalls substituierten Phenylrest bedeutet.

7. Mittel nach Anspruch 6 enthaltend als Wirkstoff eine Verbindung der Formel I, worin der Phenylkern in 2- und 6-Stellung anders als durch Wasserstoff substituiert ist.

8. Mittel nach Anspruch 7 enthaltend als Wirkstoff eine Verbindung der Formel I, worin der Substituent in der 2-Stellung des Phenylkerns eine Methylgruppe ist, und worin die Substituenten $R_1$ bis $R_4$ zusammen nicht mehr als 6 Kohlenstoffatome enthalten.

9. Mittel nach Anspruch 2 enthaltend als Wirkstoff eine Verbindung der Formel I, worin der Substituent $R_5$ den (Thio)Lactonrest bedeutet.

10. Mittel nach Anspruch 9 enthaltend als Wirkstoff eine Verbindung der Formel I, worin $R_6$ Methyl oder Aethyl ist und im Lactonring X Sauerstoff bedeutet.

11. Mittel nach Anspruch 10 enthaltend als Wirkstoff eine Verbindung der Formel I, worin Ar den gegebenenfalls substituierten Phenylrest bedeutet.

12. Mittel nach Anspruch 10 enthaltend als Wirkstoff eine Verbindung der Formel I, worin Ar den substituierten Naphthylrest bedeutet.

13. Mittel nach Anspruch 11, dadurch gekennzeichnet, dass der Phenylkern in 2- und 6-Stellung anders als durch Wasserstoff substituiert ist.

14. Mittel nach Anspruch 13, dadurch gekennzeichnet, dass $R_1$ Methyl bedeutet und dass die Substituenten $R_1$ bis $R_4$ zusammen nicht mehr als 6 Kohlenstoffatome enthalten.

15. Mittel nach Anspruch 2, enthaltend als Wirkstoff N-(1-Methoxycarbonyläthyl)-N-[(N'-methyl-N'-trichlormethylthio-sulfamoyloxy)-acetyl]-2,6-dimethylanilin.

16. Mittel nach Anspruch 2, enthaltend als Wirkstoff 3-[N-(N'-Dichlorfluormethylthio-N'-äthyl-sulfamoyloxy)-acetyl-N-(2-methylnaphthyl)--amino]-tetrahydrofuranon.

17. Mittel nach Anspruch 1, enthaltend als Wirkstoff eine Verbindung der Formel I, worin $R_7$ den 1,1,2,2-Tetrachloräthylrest bedeutet, Ar eine substituierte Phenylgruppe darstellt, in der $R_1$ Methyl ist, und $R_5$ die Gruppe $-CH(CH_3)-CO-X-CH_3$ bedeutet, in der X Sauerstoff oder Schwefel ist.

18. Mittel nach Anspruch 17, enthaltend als Wirkstoff N-(1-Methoxycarbonyläthyl)-N-[(N'-methyl-N'-1,1,2,2-tetrachloräthylthiosulfamoyloxy)-acetyl]-2,6-dimethylanilin.

19. Verfahren zur Herstellung der Verbindungen der Formel I, gemäss Anspruch 1, dadurch gekennzeichnet, dass man ein Sulfamoyloxy-acetamid der Formel II

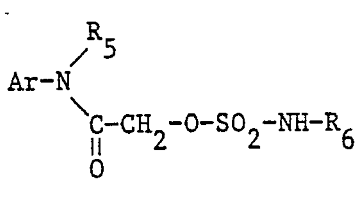

(II)

in Gegenwart einer Base mit einem Sulfenylchlorid der Formel III

$$R_7\text{-S-Cl} \qquad \text{(III)}$$

worin Ar und $R_5$ bis $R_7$ die unter Formel I angegebene Bedeutung haben, umsetzt.

20. Verfahren zur Herstellung der Verbindungen der Formel I, gemäss Anspruch 1, dadurch gekennzeichnet, dass man ein Amin der Formel IV

$$Ar\text{-NH-}R_5 \qquad \text{(IV)}$$

gegebenenfalls in Gegenwart einer Base mit einem Hydroxyessigsäure-Derivat der Formel V

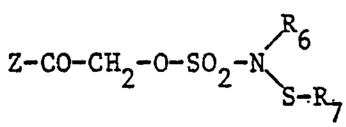

(V)

umsetzt, worin Ar und $R_5$ bis $R_7$ die unter Formel I angegebene Bedeutung haben und Z ein aktivierender Substituent ist.

21. Verwendung von Verbindungen der in Anspruch 1 definierten Formel I im Pflanzen- oder Vorratschutz zur Bekämpfung schädlicher Pilze und Bakterien.

22. Verwendung von Verbindungen der im Anspruch 2 definierten Formel I zur Bekämpfung phytopathogener Pilze und Hefen.

0050095

Europäisches Patentamt

# EUROPÄISCHER RECHERCHENBERICHT

Nummer der Anmeldung

EP 81 81 0400

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| DA | DE - A - 2 947 295 (CIBA-GEIGY)<br>* Ansprüche *<br>---- | 1-24 |

**KLASSIFIKATION DER ANMELDUNG (Int Cl.³)**

C 07 C 161/00
C 07 D 307/32
333/36
A 01 N 41/00
43/08
43/10

**RECHERCHIERTE SACHGEBIETE (Int Cl.³)**

C 07 C 161/00
C 07 D 307/32

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 18-01-1982 | GAUTIER |